# EUROPEAN PATENT APPLICATION

(11) **EP 2 386 240 A2**
(43) Date of publication of application: **16.11.2011**
(21) Application number: 11165803.5
(22) Date of filing: 12.05.2011
(51) Int. Cl.: A61B 1/00

(54) **Sheath for protecting endoscope probe**

(30) Priority: 12.05.2010 GB 1007920
(71) Applicant: Q Park Medical Limited, Stroud Gloucestershire GL5 5HT (GB)
(72) Inventor: Cant, Neil, Hucclecote, Gloucestershire GL5 5HT (GB)
(74) Representative: Ribeiro, James Michael

(57) **Abstract**

A sheath (1) for protecting an endoscope probe projecting from an endoscope head. The sheath (1) comprises: an elongate tube (1) with a bore for receiving the endoscope probe; a clamp (5) at or near a proximal end of the sheath for gripping the endoscope head or the endoscope probe; and a window (3) which is bonded to a distal end of the elongate tube. The window (3) is formed from an elastomeric material. An endoscope probe is inserted into the bore of the elongate tube (1) so that it engages the window (3) , and a force is applied so that the window is pressed and stretched against the elongate tube. The application of force causing a wall thickness of at least part of the window to decrease. The clamp (5) comprises a resilient member (4) with an elongate opening which can be opened to admit the endoscope probe by compressing the resilient member (4) in line with the length of the elongate opening.

## Description

### FIELD OF THE INVENTION

The present invention relates to a sheath for protecting an endoscope probe, a method of protecting an endoscope probe, and a method of manufacturing a window for an endoscope sheath.

### BACKGROUND OF THE INVENTION

A conventional sheath for protecting an endoscope probe is described in US6350231. The sheath comprises an elastic sheath with a bore for receiving the endoscope probe; a collar at a proximal end of the sheath; and an end cap at a distal end of the sheath.

The diameter and wall thickness of the elastic sheath decrease as the sheath is stretched axially over the probe. The reduction in wall thickness gives an increase in permeability of the sheath, thus increasing the chance of cross-infection. Also, the reduction in diameter means that the sheath can adhere to the probe, making it difficult to slide off.

The collar grips a tapered engagement portion of the endoscope head, and as a result the sheath must be sized precisely to match the length of the probe.

### SUMMARY OF THE INVENTION

A first aspect of the invention provides a sheath for protecting an endoscope probe projecting from an endoscope head, the sheath comprising: an elongate tube with a bore for receiving the endoscope probe; a clamp at or near a proximal end of the sheath for gripping the endoscope head or the endoscope probe; and a window which is bonded to a distal end of the elongate tube, wherein the window is formed from an elastomeric material.

A second aspect of the invention provides a method of protecting an endoscope probe projecting from an endoscope head using a sheath, the sheath comprising an elongate tube with a bore, a clamp at or near a proximal end of the sheath, and a window which is bonded to a distal end of the elongate tube, the method comprising: inserting the endoscope probe into the bore of the elongate tube so that it engages the window; applying a force so that the window is pressed and stretched against the elongate tube, the application of force causing a wall thickness of at least part of the window to decrease to a greater extent than the elongate tube; and retaining the force by gripping the endoscope probe or the endoscope head with the clamp.

A third aspect of the invention provides a method of protecting an endoscope probe projecting from an endoscope head using a sheath, the sheath comprising an elongate tube with a bore, a clamp at or near a proximal end of the sheath, and a window which is bonded to a distal end of the elongate tube, the method comprising: inserting the endoscope probe into the bore of the elongate tube so that it engages the window; and applying a force so that the window is pressed and stretched against the elongate tube, the application of force causing a wall thickness of at least part of the window to decrease by more than 30%, and preferably by more than 50%; and retaining the force by gripping the endoscope probe or the endoscope head with the clamp.

Forming the window from an elastomeric material such as polyurethane makes the window flexible and resilient, making it particularly suitable for use in the method of the second and/or third aspect of the invention.

Preferably the application of force causes the window to deform from an unstretched shape in which it does not match the shape of the probe to a stretched state in which it conforms with the shape of the probe.

The window and the elongate tube may be formed from material with the same elastic modulus, or the material forming the elongate tube may have a higher elastic modulus than the material forming the window, enabling the window to flex more easily than the elongate tube.

The window may be bonded to the elongate tube by cobonding (in which the window cures in contact with the tube); cocuring (in which the window and tube both cure in contact with each other); secondary bonding (in which an adhesive is used to bond the parts together); or any other suitable bonding process.

Preferably the window is more transparent than the elongate tube, at least when the sheath is fitted on an endoscope probe. This difference in transparency may be achieved by the window and the tube having a different wall thickness, surface finish, and/or material composition. For instance in a preferred embodiment the window and elongate tube are both made from the same base elastomeric material, but the sheath has an added slip agent (which makes it slightly opaque relative to the window) and a textured surface finish on both the inside and outside of the tube (which also make it slightly opaque relative to the window).

Preferably the elongate tube comprises a flexible tube which forms a majority of the length of the elongate tube. The window may be bonded directly to this flexible tube, but more preferably the elongate tube further comprises a distal fitting which carries the window and is fitted to a distal end of the flexible tube. Preferably the distal fitting and the flexible tube are formed from different materials, and the material forming the flexible tube has a lower elastic modulus than the material forming the distal fitting.

The window may be flat, or may have a preformed curved shape, such as a semi-spherical shape. Preferably the curved shape of the window is concave on the inside and convex on the outside.

The clamp may be any form of device suitable for gripping the endoscope probe or the endoscope head. For instance it may be a tapered resilient collar which engages a tapered part of the endoscope head, as described for example in US6350231. However more preferably the clamp comprises a resilient member with an elongate opening which can be opened to admit the endoscope probe by compressing the resilient member in line with the length of the elongate opening.

A fourth aspect of the invention provides a sheath for protecting an endoscope probe, the sheath comprising: an elongate tube with a bore for receiving the endoscope probe; a clamp at or near a proximal end of the sheath for gripping the endoscope probe; and a window at a distal end of the sheath, wherein the clamp comprises a resilient member with an elongate opening which can be opened to admit the endoscope probe by compressing the resilient member in line with the length of the elongate opening.

A fifth aspect of the invention provides a method of protecting an endoscope probe projecting from an endoscope head using a sheath, the endoscope probe having parallel sides which extend along its length, the sheath comprising an elongate tube with a bore, a clamp at or near a proximal end of the sheath; and a window at a distal end of the sheath, the method comprising inserting the endoscope probe into the bore of the elongate tube so that it engages the window; applying a force so that the window is pressed against the elongate tube; and retaining the force by gripping the parallel sides of the endoscope probe with the clamp.

The clamp of the fourth aspect of the invention is particularly suited for use in the method of the fifth aspect. This enables the sheath to be fitted to any length of probe, as long as the probe is longer than the sheath. Also, it prevents an excessive amount of stretch in the window, so it is particularly beneficial in combination with the first, second or third aspects of the invention.

The window may be formed integrally with the elongate tube, or may be mechanically fitted to the sheath, but more preferably the window is bonded to a distal end of the elongate tube as in the first, second and third aspects of the invention.

Preferably the clamp has a protrusion on each side, in line with the long axis of the opening. These protrusions can be used as grips for the finger and thumb.

Preferably the long axis of the opening extends transverse to the long axis of the elongate tube. The opening may also have a second long axis which is parallel with the long axis of the elongate tube.

The opening may be a slit which becomes closed when the clamp is in its relaxed uncompressed state, or more typically it is a slot which remains partially open when the clamp is in its relaxed uncompressed state.

Preferably the clamp is a clamp fitting which is formed separately from the elongate tube, although conceivably it could be formed integrally with the elongate tube.

Preferably the clamp fitting is formed as a single piece of resilient material.

Preferably the sheath further comprise a connector with a proximal end attached to the clamp fitting and a distal end attached to the tube, wherein the connector and the clamp fitting are formed from different materials, the material forming the connector having a higher elastic modulus than the material forming the clamp fitting.

A further aspect of the invention provides a method of manufacturing a window for an endoscope sheath, the method comprising applying a film of liquid to a mandrel and a bond part; curing the film of liquid so that it bonds to the bond part; and removing the mandrel, leaving the cured film in place.

The bond part may be a distal end of the endoscope sheath, or it may be a connector which is fitted to the distal end of the endoscope sheath, either before or after the window is bonded to the connector.

### BRIEF DESCRIPTION OF THE DRAWINGS

Embodiments of the invention will now be described with reference to the accompanying drawings, in which:
Figure 1 is an exploded view of an endoscope sheath;
Figure 2 shows the sheath in assembled form;
Figure 3 is a side view of the clamp fitting, with hidden detail shown in dashed lines;
Figure 4 is a front view of the clamp fitting, with hidden detail shown in dashed lines;
Figure 5 is a sectional view of the clamp fitting, with hidden detail shown in dashed lines;
Figure 6 is a bottom view of the clamp fitting;
Figure 7 is a plan view of the clamp fitting;
Figure 8 shows the proximal end of the sheath fitted to an endoscope probe;
Figure 9 shows a mandrel and distal fitting being dipped into a bath of liquid elastomer;
Figure 10 is a sectional view of the distal fitting and window;
Figure 11 is a sectional view showing the distal end of the sheath fitted to an endoscope probe. and
Figure 12 is a schematic view showing the window stretching to conform to an angled endoscope tip.

### DETAILED DESCRIPTION OF EMBODIMENT(S)

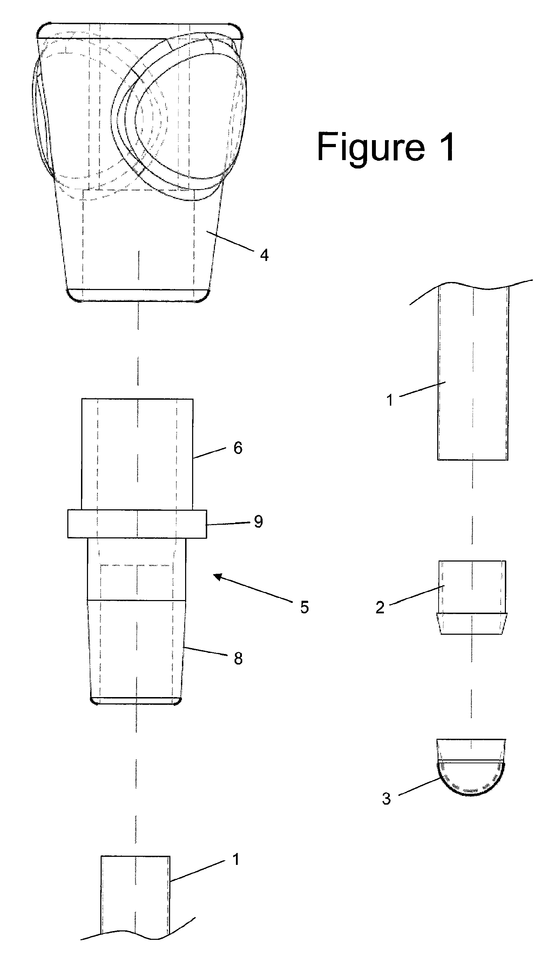

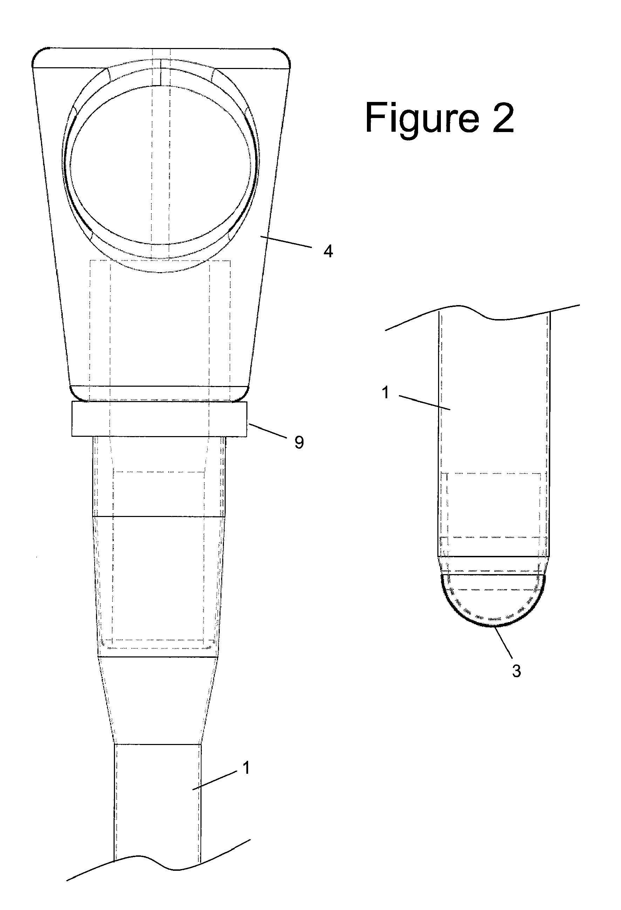

An assembled sheath shown in Figure 2 is formed by a kit of parts shown in Figure 1. The parts comprise a flexible elongate polyurethane tube 1 which forms a majority of the length of the sheath, a distal fitting 2 which carries a window 3, a clamp 4 at a proximal end of the sheath, and a clamp connector 5.

The tube 1 is manufactured by a process of extrusion as follows.
1. A batch of pellets of thermoplastic polyurethane is mixed with a batch of pellets of slip agent.
2. The mixture of pellets is fed into an extrusion chamber.
3. The extrusion chamber contains a helical worm screw which pushes the pellets along the length of the extrusion chamber.
4. The pellets are progressively heated as they move along the chamber, causing them to melt and become fully mixed.
5. The liquid mixture is then extruded from the extrusion chamber onto a pin and die tool. The pin and die tool has a pin running along its axis, and a cylindrical die surrounding the pin and spaced from it to form an annular extrusion chamber.
6. The mixture is extruded along the annular extrusion chamber and progressively cools. Thus the outer surface of the pin defines an inner diameter of the tube 1, and the inner surface of the die defines its outer diameter.
7. Once the tube has fully cooled, it is cut to size.

An example of a suitable mixture is:
- 95% by weight of Estane (R) 58311 Nat 022, supplied by Lubrizol Corporation. This is an aromatic polyether-based themoplastic polyurethane, provided in spherical pellet form; and
- 5% by weight of Estane (R) T5318 NAT 019, supplied by Lubrizol Corporation. This is an aromatic, polyether TPU based, mould release masterbatch, provided in cylindrical pellet form.

The clamp connector 5 is formed by injection moulding as a single piece from a rigid material such as PVC. The clamp connector 5 comprises a tubular proximal end 6 which is received within a bore 7 of the clamp 4, a tapered distal end 8 which is received within the flexible tube 1, and an annular flange 9 between the proximal and distal ends 6,8.

The clamp connector 5 is attached to the tube 1 by the following process.
1. A pair of jaws is used to open up the proximal end of the tube 1.
2. The flared proximal end of the tube 1 is fitted onto the distal end 8 of the connector 4 until the tube 1 engages the annular flange 9.
3. A bead of Loctite (TM) 454 instant adhesive gel is applied around the interface between the tube 1 and the annular flange 9.

The proximal end 6 of the connector 5 is received as an interference fit within the bore 7 of the clamp 4, and pushed into place until the flange 9 engages the bottom of the clamp 4 as shown in Figure 2.

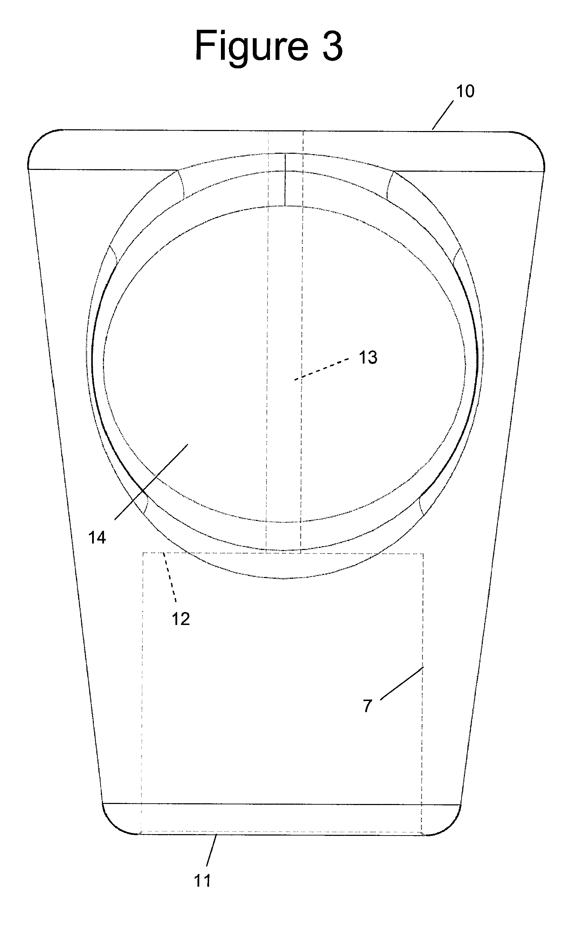

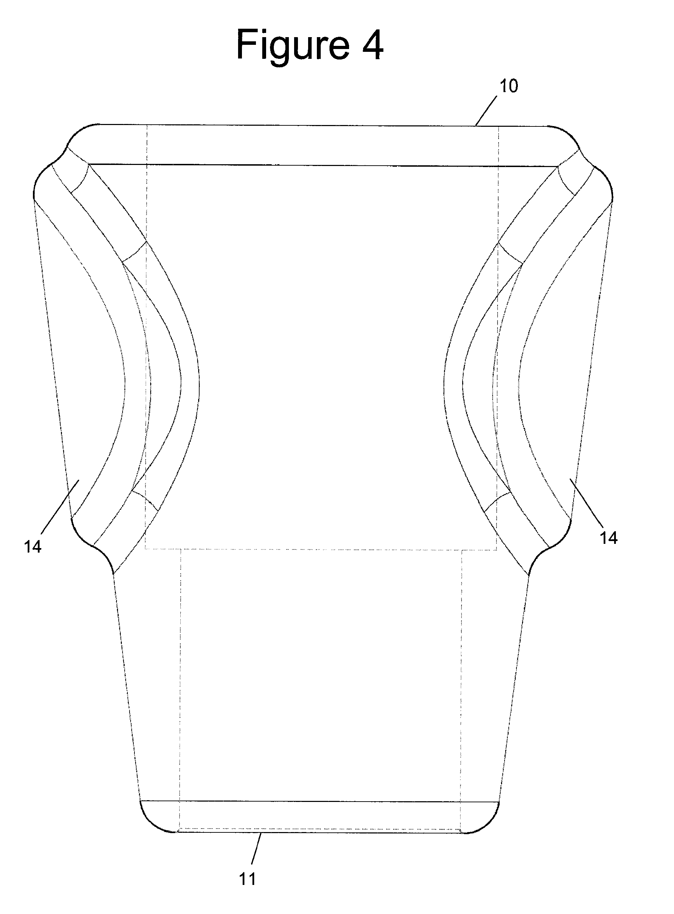

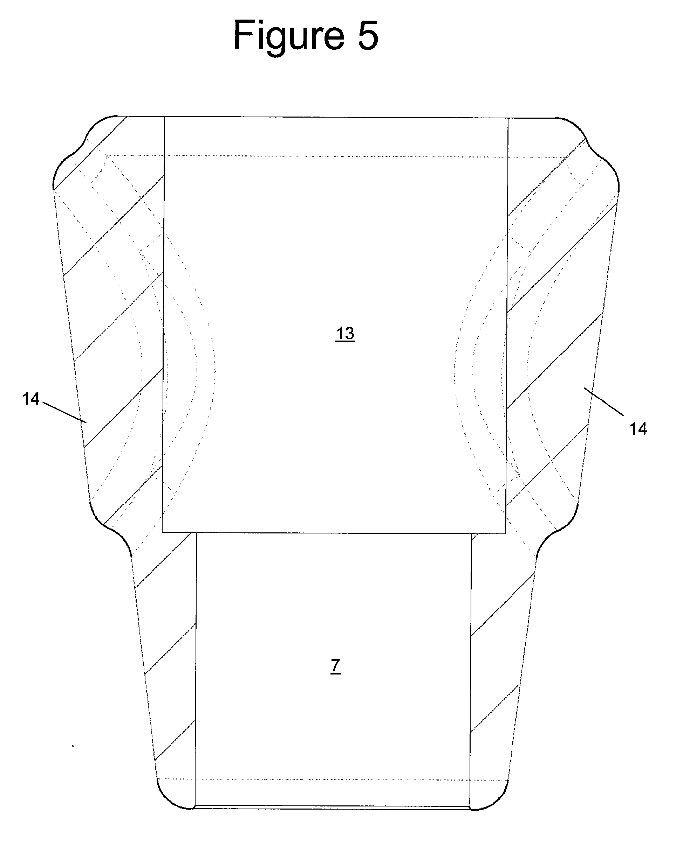

The clamp 4 is shown in detail in Figures 3-7. The clamp 5 is formed as a single piece by injection moulding from a soft resilient elastomeric material such as silicone. For example the clamp 4 may be formed from a liquid silicone rubber such as PR 415/40 supplied by Primasil Silicones Ltd of Herefordshire, UK (www.primasil.com). PR 415/40 is a Platinum catalysed liquid silicone rubber with a Shore hardness of 40, tensile strength of 10.6 MPa, elongation at break >500% and rebound resilience of 55%. It is press-cured for 5 minutes at 165°C and post-cured for 4 hours at 200 °C.

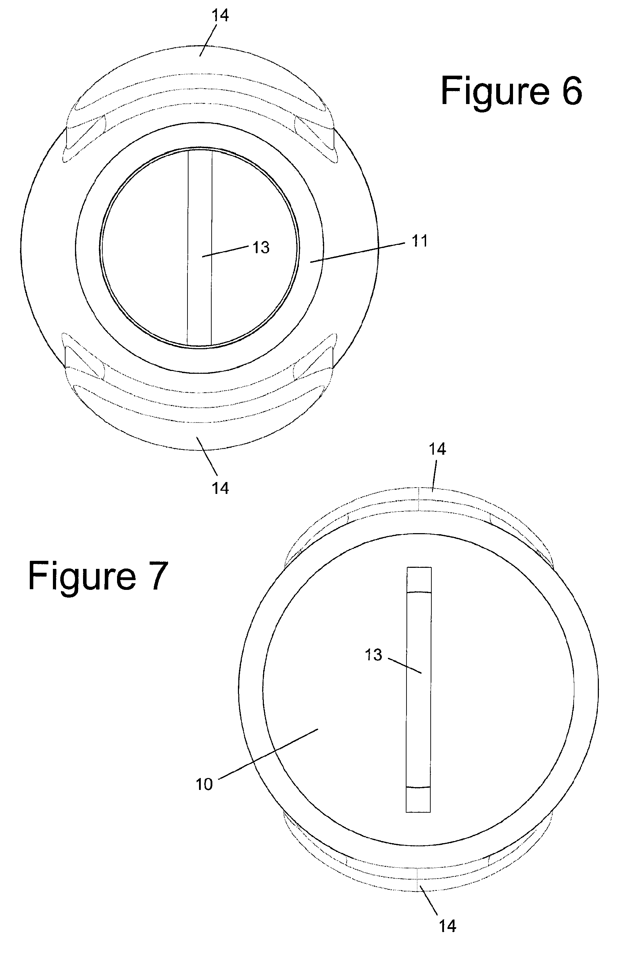

The clamp 4 has a relatively wide upper end 10 and a relatively narrow lower end 11. The bore 7 has a ceiling 12 shown in dashed line in Figure 3. A slot 13 extends between the bore 7 and the upper end 10 of the clamp. The slot 13 is elongate when viewed in line with the long axis of the sheath, (i.e. when viewed in plan as in Figure 7 or from below as in Figure 6). The slot 13 is also elongate when viewed transverse to the long axis of the sheath as in Figure 3. The clamp 4 has a protrusion 14 on each side which form a pair of finger grips, in line with the transverse long axis of the slot 13 as shown most clearly in Figure 6.

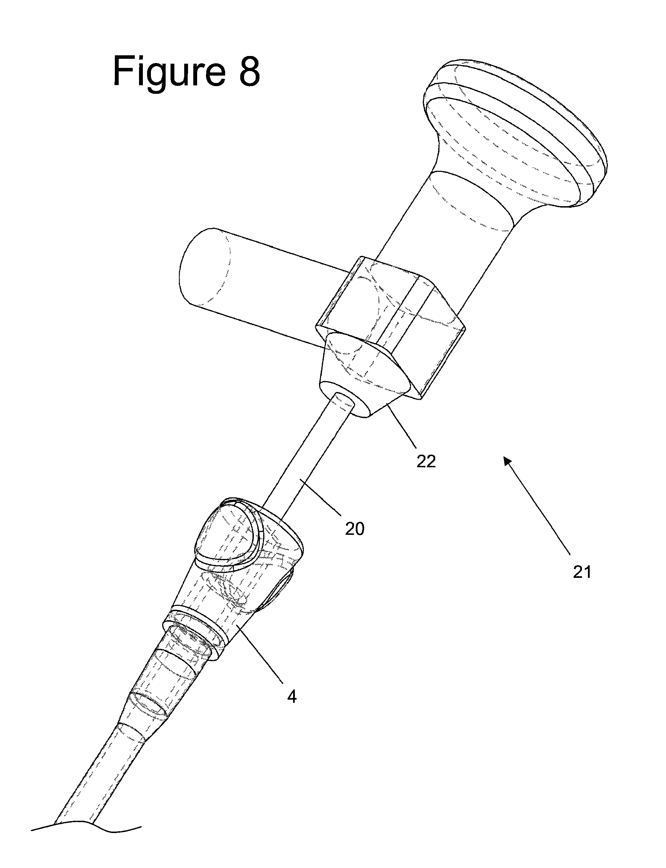

Figure 8 shows the sheath fitted to an endoscope probe 20. The endoscope probe 20 is a cylindrical tube with parallel sides which extend along its length to a distal end not shown in Figure 8. The probe 20 may be rigid or flexible. The probe 20 extends from an endoscope head 21 with a frustoconical headpiece 22. In a conventional arrangement as described in, for example, US6350231, the headpiece 22 is gripped by the endoscope sheath. By contrast, the clamp 4 grips the parallel sided probe 20 instead of the furstoconical headpiece 22. As a result the sheath can be fitted to any length of probe 20, as long as the probe 20 is longer than the sheath.

The slot 13 in the clamp 4 is opened up to admit the endoscope probe 20 by holding the clamp between thumb and index finger with the finger grips 14, and squeezing the thumb and index finger together. This compresses the clamp 4 in line with the transverse long axis of the slot 13 which causes the slot 13 to open up. After the distal end of the probe has been pushed down through the full depth of the slot, the finger grips 14 can be released and the clamp springs back towards its original uncompressed shape so that the walls of the slot 13 securely grip the probe 20, although the grip is sufficiently light to enable the probe 20 to be pushed down further into the sheath until the distal end of the probe engages the window 3.

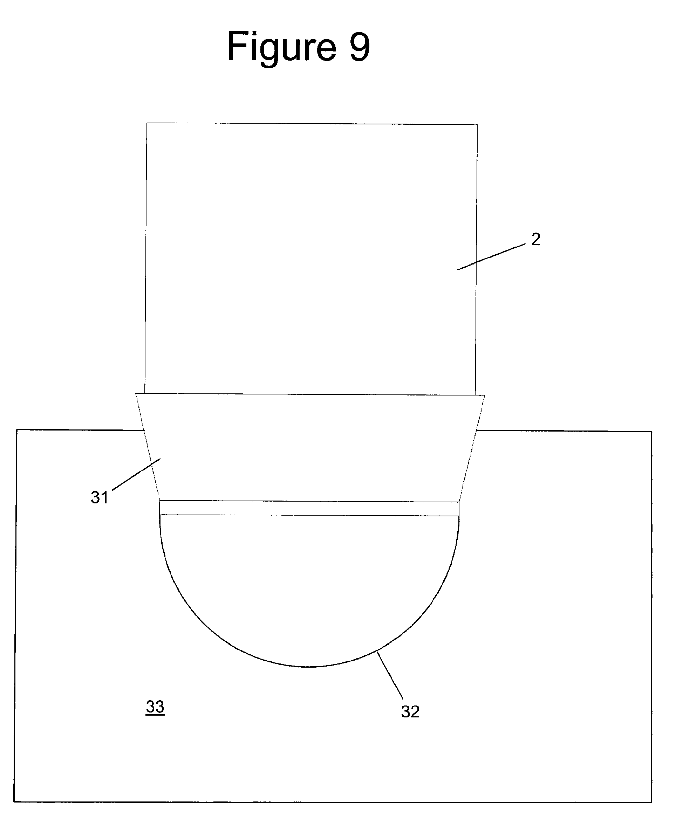

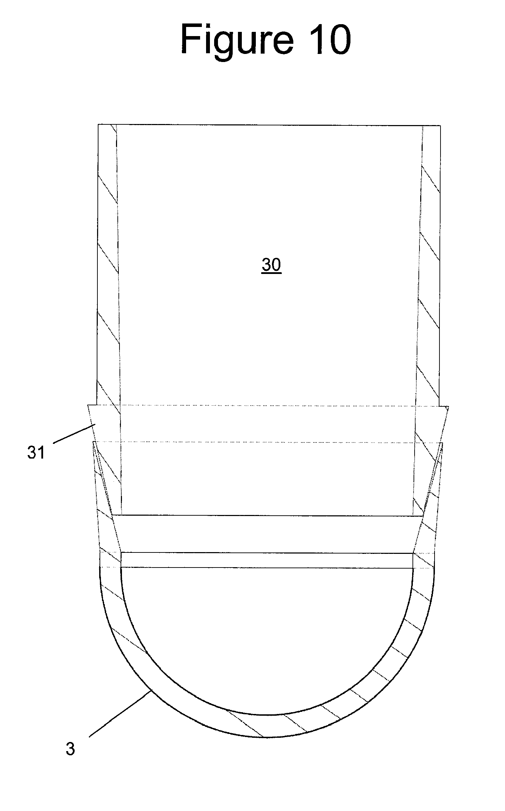

The distal end of the sheath will now be described with reference to Figures 9-11. As shown in Figure 1, the distal end of the sheath comprises a fitting 2 and a window 3. The fitting 2 is formed as a single injection moulded piece of a relatively stiff polymeric material such as "K-Resin" (TM) styrene butadiene copolymer. The fitting 2 has a bore 30 shown in Figure 10, and a tapered annular flange 31 at its distal end. The window 3 is formed and bonded to the fitting 2 by the following process.
1. A glass mandrel with a semi-spherical tip 32 is pushed through the bore 30 of the fitting 2 (either before or after the fitting 2 has been attached to the tube 1) so that it protrudes from the fitting as shown in Figure 9.
2. A bath of liquid elastomer 33 is prepared by mixing 80% by weight of Estane (R) 58311 Nat 022 (i.e. the same polyurethane material used to form the tube 1) and 20% by weight of methlyethylketone solvent UN1193 from Cotswold Chemicals & Lubricants Ltd of Gloucester, UK; and heating them at 40°C for a maximum of 2 hours so the Estane pellets liquify.
3. The mandrel 32 and a portion of the annular flange 31 are dipped into the bath of liquid elastomer 33 as shown in Figure 9.
4. The mandrel 32 and the fitting 2 are removed from the bath, leaving a film of liquid elastomer on the mandrel and the annular flange 31.
5. The methlyethylketone solvent evaporates at room temperature, leaving a cured film of substantially pure polyurethane which is cobonded to the annular flange 31.
6. The cured film does not bond to the mandrel 32 since the mandrel is formed from smooth glass. This enables the mandrel to be removed, leaving the cured film 3 in place as shown in Figure 10.

Before or after the window 3 has been formed as described above, the fitting 2 is attached to the tube 1 by the following process:
1. The proximal end of the fitting 2 is dipped into methylcyclohexanone solvent which forms a thin film on the dipped surfaces of the cap.
2. The tube 1 is pushed onto the proximal end of the fitting 2 until it engages the flange 31. Note that the inner diameter of the tube 2 is matched with the outer diameter of the fitting 2 so that little or no stretching of the tube is required as it is pushed onto the cap.
3. The methylcyclohexanone dissolves and fuses together the contacting surfaces, and evaporates from any other surfaces.

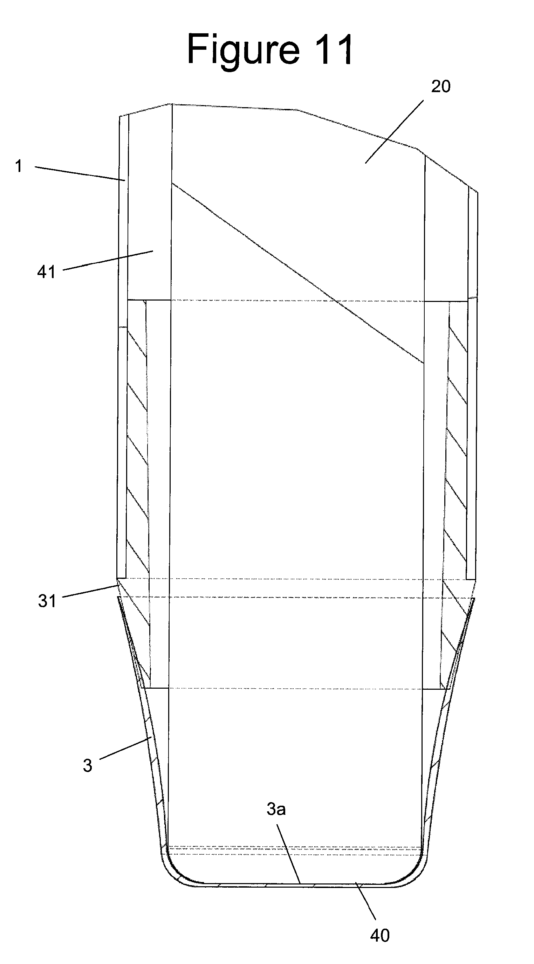

After the endoscope probe 20 has been inserted into the clamp 4 as described above with reference to Figure 8, the probe 20 is pushed down further into the bore of the tube 1, with the slip agent in the material forming the tube 1 preventing the parts from sticking. Referring to Figure 11, the distal end 40 of the probe 20 carries a lens (not shown). As the distal end 40 engages the window 3 it is pushed down further so that the window is pressed against the elongate tube. This ensures an intimate contact between the window and the lens at the distal end 40. The force is retained by the action of the clamp 4 which grips the endoscope probe 20.

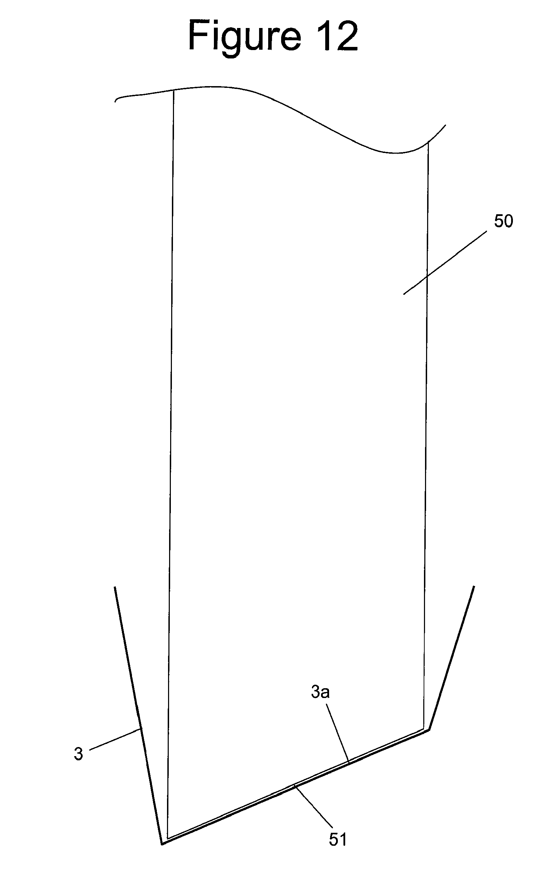

As well as pressing the window against the probe, the force also causes the window 3 to stretch and deform so that is conforms with the shape of the distal end 40, as shown in Figure 11. Thus in the example of Figure 11 the window deforms from an unstretched preformed semi-spherical shape, to a stretched shape in which the central part 3a of the window which engages the distal end 40 of the probe is flat and at right angles to the length of the probe. Figure 12, on the other hand, is a schematic view showing the window 3 stretching to conform to an angled tip 51 of an endoscope 50. Note that Figure 12 is schematic only, and omits the fitting 2, tube 1 etc.

In its unstretched form shown in Figure 10 the wall thickness of the window 3 is typically relatively constant, and of the order of 0.3-0.4mm. In its stretched form shown in Figure 11 the wall thickness of the central part 3a engaging the distal end is of the order of O.lmm. Therefore the application of force causes a wall thickness of at least the central part 3a to decrease by 67%-75%. This reduction of wall thickness increases the transparency of the window, without significantly increasing the risk of permeability of the sheath since the window has a relatively small area. In comparison, the tube 1 does not stretch to any significant extent and maintains a wall thickness of the order of 0.13mm so there is no change in permeability through the tube 1.

The lack of stretch in the tube 1 maintains a gap 41 between the tube 1 and the probe 20. This gap 41 prevents the tube 1 from sticking to the probe 20, making it easy for a user to remove the sheath from the probe 20 after use.

Removal of the sheath is also assisted by the distal fitting 2, particularly if the window 3 has stuck to the distal end of the probe 20. The polyurethane material forming the tube 1 has a lower elastic modulus than the polystyrene material forming the distal fitting 2. As a result, the fitting 2 is relatively stiff so it can be easily gripped between thumb and index finger to pull the distal end of the sheath off the distal end of the probe 20.

Although the window 3 and elongate tube 1 are both made from the same base elastomeric material, the sheath is more opaque than the window because it has an added slip agent (which makes it slightly opaque relative to the window) and a textured surface finish on both the inside and outside of the tube (which also make it slightly opaque relative to the window which has a smooth surface finish).

Although the invention has been described above with reference to one or more preferred embodiments, it will be appreciated that various changes or modifications may be made without departing from the scope of the invention as defined in the appended claims.

## Claims

1. A sheath for protecting an endoscope probe projecting from an endoscope head, the sheath comprising:
an elongate tube with a bore for receiving the endoscope probe;
a clamp at or near a proximal end of the sheath for gripping the endoscope head or
the endoscope probe; and
a window which is bonded to a distal end of the elongate tube,
wherein the window is formed from an elastomeric material.

2. The sheath of claim 1 wherein the material forming the elongate tube has a higher elastic modulus than the material forming the window.

3. The sheath of any preceding claim wherein the window has a preformed curved shape.

4. The sheath of any preceding claim wherein the clamp comprises a resilient member with an elongate opening which can be opened to admit the endoscope probe by compressing the resilient member in line with the length of the elongate opening.

5. A sheath for protecting an endoscope probe, the sheath comprising:
an elongate tube with a bore for receiving the endoscope probe;
a clamp at or near a proximal end of the sheath for gripping the endoscope probe;
and
a window at a distal end of the sheath,
wherein the clamp comprises a resilient member with an elongate opening which can be opened to admit the endoscope probe by compressing the resilient member in line with the length of the elongate opening.

6. The sheath of claim 4 or 5 wherein the clamp has a protrusion on each side, in line with the long axis of the opening.

7. The sheath of claim 5 or 6 further comprising a connector with a proximal end attached to the clamp fitting and a distal end attached to the tube, wherein the connector and the clamp fitting are formed from different materials, the material forming the connector having a higher elastic modulus than the material forming the clamp fitting.

8. A method of protecting an endoscope probe projecting from an endoscope head using a sheath, the sheath comprising an elongate tube with a bore, a clamp at or near a proximal end of the sheath, and a window which is bonded to a distal end of the elongate tube, the method comprising: inserting the endoscope probe into the bore of the elongate tube so that it engages the window; applying a force so that the window is pressed and stretched against the elongate tube, the application of force causing a wall thickness of at least part of the window to decrease to a greater extent than the elongate tube; and retaining the force by gripping the endoscope probe or the endoscope head with the clamp.

9. The method of claim 8 wherein the application of force causes a wall thickness of at least part of the window to decrease by more than 30%, and preferably by more than 50%.

10. A method of protecting an endoscope probe projecting from an endoscope head using a sheath, the sheath comprising an elongate tube with a bore, a clamp at or near a proximal end of the sheath, and a window which is bonded to a distal end of the elongate tube, the method comprising: inserting the endoscope probe into the bore of the elongate tube so that it engages the window; and applying a force so that the window is pressed and stretched against the elongate tube, the application of force causing a wall thickness of at least part of the window to decrease by more than 30%, and preferably by more than 50%; and retaining the force by gripping the endoscope probe or the endoscope head with the clamp.

11. The method of claim 8, 9 or 10 wherein the application of force causes the window to deform from an unstretched shape in which it does not match the shape of the probe to a stretched state in which it conforms with the shape of the probe.

12. The method of any of claims 8 to 12 wherein the endoscope probe has parallel sides which extend along its length, and the method further comprises retaining the force by gripping the parallel sides of the endoscope probe with the clamp.

13. A method of protecting an endoscope probe projecting from an endoscope head using a sheath, the endoscope probe having parallel sides which extend along its length, the sheath comprising an elongate tube with a bore, a clamp at or near a proximal end of the sheath; and a window at a distal end of the sheath, the method comprising inserting the endoscope probe into the bore of the elongate tube so that it engages the window; applying a force so that the window is pressed against the elongate tube; and retaining the force by gripping the parallel sides of the endoscope probe with the clamp.

14. A method of manufacturing a window for an endoscope sheath, the method comprising applying a film of liquid to a mandrel and a bond part; curing the film of liquid so that it bonds to the bond part; and removing the mandrel, leaving the cured film in place.

15. The method of claim 14 wherein the film of liquid is applied by dipping the mandrel and bond part into a bath of liquid.
